Europäisches Patentamt

European Patent Office

Office européen des brevets

(11) Publication number : **0 486 443 A1**

# EUROPEAN PATENT APPLICATION

(21) Application number : **91830495.7**

(22) Date of filing : **12.11.91**

(51) Int. Cl.⁵ : **G01N 33/18**

(30) Priority : **15.11.90 IT 4847990**

(43) Date of publication of application :
**20.05.92 Bulletin 92/21**

(84) Designated Contracting States :
**AT BE CH DE DK ES FR GB GR LI LU NL SE**

(71) Applicant : **GENESIS S.r.L.**
**Via Reno 35**
**I-00198 Roma (IT)**

(72) Inventor : **Antonini, Giovanni**
**Loc. Pedalicchio**
**I-01032 Caprarola (VT) (IT)**
Inventor : **D.sa Piera Valenti**
**Via della Pelliccia 11**
**I-00153 Roma (IT)**

(74) Representative : **Massari, Marcello**
**Studio M. Massari S.r.l. 23, Via Fontanella**
**Borghese**
**I-00186 Roma (IT)**

(54) **Method of detecting toxic compositions in water by monitoring the metabolism of selected living cells as microorganisms.**

(57)  The method is based upon the time detection of some biochemical parameters of microbial metabolism such as acidification of the environment, production of carbon dioxide, increase of turbidity, and decrease of dissolved oxygen. Such parameters are detected in a thermostated cell containing particular microorganisms and a composition having a toxic effect. The detected parameters have a different course with respect to those detected in a thermostated control cell where the toxic composition is left out. The decrease of the values of the normal microbial metabolism is proportional to the amount of toxic composition. Such a method can then be used to detect without the chemical analysis of the chassic methods any amount of a toxic composition in water. To this end microorganisms properly selected for their sensitivity to different toxic compositions are used.

EP 0 486 443 A1

The present invention relates to the detection of toxic compositions in water by monitoring the metabolism of living cells as e.g. microorganisms selected for their sensitivity to classes of toxic compositions.

Chemical pollution is a phenomenon which is so worth noting today that people should deal sistematically therewith. Chemical pollution (poison gases, pesticides, a.s.o.) are being detected in water, soil or atmosphere for years by the classic analytical technology.

However, disadvantageous features of such detection are:

– the time discontinuity of the measurements which are usually carried out on request of the competent authorities in consequence of questionable pollutions or manifest, almost irreversible environmental damage;

– a long delay between sampling, measurement and analysis of data;

– higher cost for laboratory equipment, reactants, and the necessary qualified staff;

– lack of standardized analysis methods for several toxic compositions.

This invention discloses methods of biologically detecting toxic compositions which can firstly be used for an automatic continuous monitoring system of polluting agents. The analysis effected by the methods described afterwards can be automatically performed at times and cost lower than those of the analysis performed by classical methods.

In addition, one advantage of the method described later on over the classic analytical method is that the same adapted to detect compositions having a harmful action on the living cells apart from the chemical structure of the molecule. The described methods allow the biological detector to be throughly integrated with microelectronic data collection units by developing in economically convenient way biosensors for toxic compositions.

It is then to point out that the procedure of detecting toxic compositions in a fluid described afterwards has been developed to be specifically used for manufacturing an automated biosensor for toxic compositions which will be of considerable importance for the networks of monitoring domestic-use water.

The experimental, biological processed protocol is part of the present invention and can be summarized as follows:

1 - Identification of the toxic compositions to be detected;

2 - Selection of microorganisms already sensitive in part to the selected compositions or having, as foreseen according to the present knowledge, a potential sensitivity to the selected toxic composition;

3 - Isolation and selection of stocks having increased sensitivity to the class of selected toxic compositions and provided by passing in succession on a plate (technique of iteration);

4 - Selection of the biochemical parameter to be used for controlling any suffering condition of the test microorganism in the course of time by determination of the metabolism and iteration rate thereof. The biochemical parameter can be selected among the detection of: acidification of a selected medium, production of carbon dioxide, increasing of turbidity, and decreasing of the dissolved oxygen. Such selection depends upon the microorganism and the metabolic way or cellular structures whose functionality has been presumably injured by the toxic composition.

5 - Correlation between amount of toxic composition and detected degree of alteration of the biochemical parameter and plotting a standard dose-effect response curve.

In the following examples there are disclosed the methods used to detect an exemplificative toxic composition: phenol. However, it should be noted that the methods used to detect such toxic composition can also be used under the same criteria and the same modality to detect any toxic composition belonging to the most varied chemical sorts such as ions, heavy metals, organic and inorganic compounds, with the only limitation that they have a harmful activity to living organisms like prokaryotes and eukaryotes.

At the same way, it should be appreciated that the genera and species of microorganisms used in the following examples are only an exemplification and any microbial genus or species belonging to prokaryotes and eukaryotes can be used provided that:

1) The microorganism is selected for its sensitivity to a particular toxic composition;

2) The metabolism or the growth of the microorganism can be made apparent through the detection of one of the following biochemical parameters: acidification of the environment medium, production of carbon dioxide, increasing of turbidity or decreasing of dissolved oxygen.

3) Such biochemical parameters can be monitored in a suitable microbial culture medium containing both microorganisms and the toxic composition to be detected.

## EXAMPLE 1

Detecting of the amount of toxic composition in a liquid by monitoring the acidification of a suitable nutrient medium containing the related toxic composition and a stock of Escherichia coli selected for its poor resistance to the chosen toxic composition.

The stock of E. coli K12 used in this example has been selected through passages in succession on a nutrient medium containing decreasing amounts of phenol selected as toxic composition to be detected.

The most sensitive colonies to the phenol have been detected by the socalled method of iteration on a plate so as to detect colonies which were not capable of developing at a phenol concentration of $10^{-5}$M. It is known that the metabolism and the growth of E. coli in a suitable nutrient medium are followed by an acidification of the medium due to the production of weak acids by the microorganism; moreover it is known that such acidification can be monitored by potentiometer methods.

The test of detecting phenol in a liquid (water) was carried out as follows:

9 parts of water, 1 part of ten-time concentrated nutrient medium for E. coli, and variable amounts of phenol with concentrations between 0 and $10^{-6}$M were put into a cell thermostated at 37°C and containing an electrode for the potentiometric detection of pH. Subsequently the stock of E. coli (up to a total amount of $10^6$ cells per ml) selected for its sensitivity to phenol as mentioned above was added. The variation of pH of the liquid contained in the reaction vessel was monitored in the course of time.

The detection of a significant acidification after 3 hours indicated the normal development of the bacterium and then the absence of the toxic composition, while the detection of a retarded acidification or the absolute lack of acidification after 12 hours indicated the presence of the toxic composition. Furthermore an approximately linear correlation between the time delay of the acidification in the presence of the toxic composition and the logarithm of the phenol concentration up to a limit of $10^{-5}$M was detected. For a concentration of phenol greater than $10^{-2}$M there was no acidification at all after 12 hours.

## EXAMPLE 2

Detection of the amount of toxic composition in a liquid by monitoring the production of carbon dioxide in a suitable nutrient medium containing the toxic composition and a stock of Saccharomyces cerevisiae selected for its poor resistance to the selected toxic composition.

The stock of S. cerevisiae KL144A used in the example was selected through passages in succession on a nutrient medium containing decreasing amounts of phenol selected as toxic composition to be detected. The most sensitive colonies to phenol were detected by the socalled method of iteration on a plate. In this way colonies which were not capable of developing at a phenol concentration of $10^{-5}$M were detected. It is known that the metabolism and the growth of S. cerevisiae in suitable nutrient mediums is followed by the production of $CO_2$ from the catabolism of glucides, and it is also known that such production can be detected by potentiometer methods.

The test of detecting phenol in a liquid (water) was carried out as follows:

9 parts of water, 1 parts of ten-time concentrated nutrient medium for S. cerevisiae, and variable amounts of phenol with concentrations between 0 and $10^{-6}$M were put into a vessel thermostated at 30°C and containing an electrode for the potentiometer detection of carbon dioxide. The stock of S. cerevisiae (up to a total amount of $10^6$ cells per ml) selected for its sensitivity to phenol as mentioned above was then added, and the production of $CO_2$ in the liquid contained in the reaction vessel was monitored in the course of time.

The detection of a significant production of carbon dioxide after 6 hours indicated the normal development of the microorganism and then the lack of any toxic composition, while the detection of a retarded production or no production at all after 12 hours indicated the presence of the toxic composition. Furthermore an approximately linear correlation between the time delay of the production of $CO_2$ in the presence of the toxic composition and the logarithm of the phenol concentration up to a limit of $10^{-5}$M was detected. For concentration greater than $10^{-2}$M there was no production of $CO_2$ at all.

## EXAMPLE 3

Detection of the amount of toxic composition in a liquid by monitoring the turbidity of a suitable nutrient medium containing the toxic composition and a stock of Streptococcus faecalis selected for its poor resistance to the chosen toxic composition.

The stock of S. faecalis 125 used in this example was selected through passages in succession on a nutrient medium containing decreasing amounts of phenol selected as toxic composition to be detected. The most sensitive colonies to phenol were detected by the method of iteration on a plate, and in this way colonies which were not capable of developing at a phenol concentration of $10^{-5}$M were detected. It is known that the multiplication of S. faecalis in suitable nutrient mediums was followed by an increase of the turbidity of the environment medium due to the increase of particulate matter in suspension (the same bacterium). It is also known that such increase in the turbidity can be monitored by an optical system formed of a light source having wavelenghts in the visible, and a photometer. The multiplication of the particulate matter in suspension (the selected bacteria) is reflected to an increase in the turbidity, thus causing a reduction of the light amount emitted by the source, which can reach the photometer which is placed such that the emitted light should pass through the medium containing the microorganisms before being analyzed by the photometer.

The test of detecting phenol in a liquid (water) was carried out as follows:

9 parts of water, 1 parts of ten-time concentrated nutrient medium for S. faecalis 125, and variable amounts of phenol with concentrations between 0 and $10^{-6}$M were put into a vessel thermostated at 37°C and containing the above described optical system. The stock of S. faecalis (up to a total amount of $10^{-6}$ cells per ml) selected for its sensitivity to phenol as mentioned above was then added, and the increase in the turbidity of the liquid contained in the reaction vessel was monitored in the course of time through the decreasing of the light received by the photometer.

The detection of a significant increase in the turbidity after 6 hours indicated the normal development of the microorganism and then the lack of any toxic composition, while the detection of a retarded turbidity or no light decreasing at all after 12 hours indicated the presence of the toxic composition. Furthermore an approximately linear correlation between the time delay at which the turbidity took place in the presence of phenol and the logarithm of the phenol concentration up to a limit of $10^{-5}$M was detected. For concentration greater than $10^{-2}$M there was no turbidity at all.

## EXAMPLE 4

Detection of the amount of toxic composition in a liquid by monitoring the amount of dissolved oxygen in a suitable nutrient medium containing the toxic composition and a stock of Candida utilis selected for its poor resistance to the chosen toxic composition.

The stock of Candida utilis A51 used in this example was selected through passages in succession on a nutrient medium containing decreasing amounts of phenol selected as toxic composition to be detected. The most sensitive colonies to phenol were detected by the method of iteration on a plate, and in this way colonies which were not capable of developing at a phenol concentration of $10^{-5}$M were detected. It is known that the metabolism and the multiplication of C. utilis in suitable nutrient mediums was followed by a decrease of dissolved oxygen in the medium as the latter is consumed by the metabolism of the microorganism. It is also known that such decrease of dissolved oxygen can be monitored in the course of time by polarographic methods. The test of detecting phenol in a liquid (water) was carried out as follows:

9 parts of water, 1 parts of ten-time concentrated nutrient medium for C. utilis 125, and variable amounts of phenol with concentrations between 0 and $10^{-6}$M were put into a vessel thermostated at 30°C and containing a polarographic detector of dissolved oxygen. The stock of C. utilis (up to a total amount of $10^6$ cells per ml) selected for its sensitivity to phenol as mentioned above was then added, and the decrease in the concentration of dissolved oxygen was monitored. The detection of a significant decrease in the dissolved oxygen after 3 hours indicated the normal development of the microorganism and then the lack of any toxic composition, while the detection of a retarded decrease or no decreasing at all of dissolved oxygen after 12 hours indicated the presence of the toxic composition. Furthermore an approximately linear correlation between the time delay at which the decrease in the dissolved oxygen took place in the presence of phenol and the logarithm of the phenol concentration up to a limit of $10^{-5}$M was detected. For phenol concentration greater than $10^{-2}$M there was no decrease in the dissolved oxygen at all.

## Claims

1. A method of detecting a toxic composition in water, wherein living cells as microorganisms sensitive to a toxic composition are monitored in the course of time by controlling the metabolic parameters in the presence or in the absence of that toxic composition.

2. The method of claim 1, wherein the microorganisms, both prokaryotes and eukaryotes, have been selected for their sensitivity to a toxic composition by the method of iteration on a plate irrespective of the chemical nature of the toxic composition.

3. The method of claims 1 and 2, wherein the. biochemical parameter used for the detection of the microbial metabolism in the course of time is the potentiometer measurement of the pH of the medium in which the toxic composition, a suitable culture medium, and the microorganisms selected for their sensitivity to said toxic composition are contained.

4. The method of claims 1 and 2, wherein the biochemical parameter used for the detection of the microbial metabolism in the course of time is the potentiometer measurement of the production of carbon dioxide in the medium in which the toxic composition, a suitable culture medium, and the microorganisms selected for their sensitivity to said toxic composition are contained.

5. The method of claims 1 and 2, wherein the biochemical parameter used for the detection of the microbial metabolism in the course of time is the photometric measurement of the turbidity, which is caused by the microbial development, of the medium in which the toxic composition, a suitable culture medium, and the microorganisms selected for their sensitivity to said toxic composition are contained.

6. The method of claims 1 and 2, wherein the biochemical parameter used for the detection of the microbial metabolism in the course of time is the polarographic measurement of the oxygen dissolved in the medium in which the toxic composition, a suitable culture medium, and the microorganisms selected for their sensitivity to the toxic composition are contained.

7. The method of claims 3, 4, 5 and 6, wherein the detection of the biochemical parameters of the microbial metabolism is effected in a thermostated reaction cell, the loading of which can be carried out both manually and automatically.

8. The method of claims 3, 4, 5, 6 and 7, wherein the alteration degree of the biochemical parameter in the course of time because of the toxic composition is related to the concentration of the toxic composition itself.

**European Patent Office**

# EUROPEAN SEARCH REPORT

Application Number

EP 91 83 0495

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 228 407 (BROWN, BOVERI & CIE AKTIENGESELLSCHAFT) <br> * the whole document * <br> --- | 1,3-8 | G01N33/18 |
| X | US-A-4 620 930 (MCDOWELL) <br> * column 3, line 10 - column 7, line 60 * <br> --- | 1,2,6 | |
| X | US-A-4 513 280 (HANNAN) <br><br> * column 2, line 28 - column 6, line 26; figures 1,2 * <br> --- | 1,3,4,7,8 | |
| X | DE-A-2 651 896 (BROWN, BOVERI & CIE, AKTIENGESELLSCHAFT) <br> * the whole document * <br> --- | 1,6 | |
| A | EP-A-0 304 406 (GENESIS S.R.L.) <br> * the whole document * <br><br> ----- | 3-7 | |
| | | | **TECHNICAL FIELDS SEARCHED (Int. Cl.5)** <br><br> G01N |

The present search report has been drawn up for all claims

| Place of search | Date of completion of the search | Examiner |
|---|---|---|
| THE HAGUE | 27 FEBRUARY 1992 | R.A.P. BOSMA |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)